# EUROPÄISCHE PATENTANMELDUNG

(11) **EP 1 457 203 A1**
(43) Veröffentlichungstag der Anmeldung: **15.09.2004**
(21) Anmeldenummer: 04004792.0
(22) Anmeldetag: 02.03.2004
(51) Int. Cl.: A61K 9/20, A61K 9/28, A61K 9/48, B65D 83/04, A61J 7/00

(54) **Feste, exakt dosierbare pharmazeutische Darreichungsformen zur Einzelausgabe aus Dosiervorrichtungen und Verfahren hierfür**

(30) Priorität: 05.03.2003 DE 10309473
(71) Anmelder: Nordmark Arzneimittel GmbH & Co.KG, 25436 Uetersen (DE)
(72) Erfinder: Moest, Thomas, Dr., 25436 Moorrege (DE); Lämmerhirt, Klaus, Dr., 25436 Moorrege (DE); Lüdemann, Jan, Dr., 25474 Bönningstedt (DE)
(74) Vertreter: Richter, Werdermann, Gerbaulet & Hofmann

(57) **Zusammenfassung**

Um eine feste, exakt dosierbare, feste pharmazeutische Darreichungsformen zur Einzelausgabe aus Dosiervorrichtungen zu schaffen, die einerseits stabil sind und jeweils einheitlich und gleichmäßig dosiert werden können, andererseits durch die Möglichkeit einer beliebigen und genauen Abteilbarkeit eine präzise optimale individuelle Dosierung für unterschiedlichste Anforderungen gestatten, ist vorgesehen, dass die Darreichungsformen kleine sphäroide Festkörper mit mindestens einem Wirkstoff bei Bedarf zusammen mit pharmazeutischen Hilfsstoffen darstellen und einen exakten Masse- und Wirkstoffgehalt aufweisen.

## Beschreibung

Die Erfindung betrifft feste, exakt dosierbare pharmazeutische Darreichungsformen zur Einzelausgabe aus Dosiervorrichtungen, die kleine sphäroide Festkörper darstellen, wobei sie mindestens einen Wirkstoff zusammen mit pharmazeutischen Hilfsstoffen umfassen und einen exakten Masse- und Wirkstoffgehalt aufweisen. Weiterhin betrifft die Erfindung Verfahren zur Herstellung der festen pharmazeutischen Darreichungsformen sowie ihre Verwendung. Solche Darreichungsformen ermöglichen eine individuelle Einzeldosierung abgestimmt auf die Anforderungen einer optimalen Patientenbehandlung in Verbindung mit Dosiervorrichtungen.

Die Herstellung von oral beziehungsweise peroral applizierbaren Arzneiformen ist aus zahlreichen Publikationen bekannt. In der Regel werden oral anwendbare Arzneimittel in Form von festen Zubereitungen oder Liquida eingesetzt. Feste Darreichungsformen von Arzneimitteln, wie z. B. Tabletten oder Kapseln, sind flüssigen Verabreichungsformen bekanntermaßen vorzuziehen, da sie in tieferen Abschnitten des Verdauungstraktes zur Wirkung kommen. Sie ermöglichen somit eine zielgerichtete Freisetzung von wirksamen Bestandteilen und dadurch eine gut regelbare Therapie. Außerdem sind feste Darreichungsformen durch eine gute Stabilität gekennzeichnet, können problemlos verpackt, gelagert und transportiert werden.

Oberstes Ziel einer Arzneimitteltherapie ist die individuelle Anpassung der Dosierung der Wirkstoffe auf
a) individuelle Erfordernisse der einzelnen Patienten und
b) die Anforderung der jeweiligen Therapiephase, in denen sich ein Patient befindet.

Das bedeutet, es wird eine Vielzahl von Dosierungen des gleichen Wirkstoffs bei der Einstellung von Patienten auf bestimmte Wirkstoffe aufgrund unterschiedlicher Dosisanforderungen, unterschiedlicher Ansprechzeiten oder auch unterschiedlicher Patientengruppen (z. B. Erwachsene und Kinder) benötigt. Weiterhin benötigen Patienten z. B. einschleichende und ausschleichende Wirkstoffdosierungen oder Tageszeiten abhängige Wirkstoffmengen (Chronopharmakologie). Gegebenenfalls ist auch die Dosisadaption an die tägliche Leistungssituation eines Patienten notwendig. Für die individuelle Einstellung von Dosierungen haben feste Darreichungsformen, wie z. B. Tabletten, den Vorteil, dass in einer präzise abgeteilten und als Tablette fixierten Masse eine genau definierte Wirkstoffdosis enthalten ist. Eine patientenindividuelle Anpassung erfolgt gegenwärtig in der Regel über die Größe der pharmazeutischen Darreichungsform oder über eine Teilung derselben.

Beide Anpassungsmöglichkeiten haben entscheidende Nachteile. Eine individuelle Anpassung über die Tablettengröße benötigt für jede Dosis jeweils eine neue, separate Zulassung. Dieses ist zum einen teuer und zum anderen auch unrentabel, da oft nicht die Mindestabsatzmenge erreicht werden kann, die für eine individuelle Produktion notwendig ist. Auch wird durch eine Erhöhung der Dosisstärken nur eine partielle Verbesserung der patientenindividuellen Dosierung erreicht, da ansonsten die Anzahl an verschiedenste Dosierungsgehalte ins Unermessliche steigen würde.

Die Teilbarkeit von Tabletten zur Anpassung der patientenindividuellen Dosierung erfüllt meist nicht die pharmazeutischen Anforderungen, die an die Qualität einer einzeldosierten Arzneiform zu stellen sind, nämlich Gleichförmigkeit des Gehalts bezogen auf das abgeteilte Teilstück.

Auf dem Pharmamarkt ist deshalb ein Trend zur Diversifikation bezüglich Dosierung und Darreichungsformen von Wirkstoffen eingetreten, die durch die steigenden Ansprüche an die Uniformität der Wirkstoffgabe bedingt ist, was aber durch teilbare Tabletten in der Regel eben nicht gewährleistet ist. Die qualitativen pharmazeutischen Mängel steigen aufgrund von Ungenauigkeitsfortsetzungen mit der Anzahl der Teilungen einer Tablette. Tabletten in ihrer herkömmlichen Anwendung sind deshalb ungeeignet, eine einfache und patientenindividuelle Wirkstoffdosierung zu realisieren.

Flüssige Formulierungen, sofern sie als Einphasensysteme oder homogen disperse Mehrphasensysteme vorliegen, besitzen im Prinzip die Möglichkeit der patientenindividuellen Dosisanpassung durch Abteilung eines definierten Volumens. Hierfür werden volumetrische Dosierhilfen (Messbecher) oder Applikatoren (Tropfer) eingesetzt. Wie bereits eingangs erwähnt, sind jedoch flüssige Darreichungsformen festen Formulierungen unterlegen. So besteht z. B. bei der Anwendung von Messbechern die Problematik, dass der Laie das Füllen eines Behältnisses bis zur Sollhöhe unter Einbeziehung des Meniskus an der Flüssigkeitsoberfläche nicht beherrscht. Auch ältere Patienten haben aufgrund verminderter Sehstärke und motorischer Einschränkung erhebliche Schwierigkeiten. Tropfer als Dosierhilfe zeigen dagegen oft erhebliche Probleme in der Handhabung (z. B. erfolgt möglicherweise kein spontanes Antropfen; die Tropfengröße ist verkehrt, wenn die Tropfen aus dem Belüftungsloch kommen; Abhängigkeit der Tropfengröße und Geschwindigkeit von dem Halten der Tropfflasche vom Füllgrad der Flasche, von der Temperatur usw.).

Da flüssige Arzneiformen grundsätzlich möglichst auf wässriger Basis hergestellt werden sollten, um Alkoholprobleme in der Bevölkerung auszuschließen und z. B. Kinder zu schützen, besteht ein großer Nachteil darin, dass sehr viele Wirkstoffe in einer flüssigen, wässrigen Phase nicht stabil sind. Die Anforderung an eine patientenindividuelle Dosierung ist somit auch durch flüssige Applikationsformen nicht ausreichend realisierbar.

Der Erfindung lag deshalb die Aufgabe zugrunde, pharmazeutische Darreichungsformen für Wirkstoffe so bereitzustellen, dass sie einerseits stabil sind und jeweils einheitlich und gleichmäßig dosiert werden können, andererseits durch die Möglichkeit einer beliebigen und genauen Abteilbarkeit eine präzise optimale individuelle Dosierung für unterschiedlichste Anforderungen gestatten und darüberhinaus ein Herstellungs- und Verarbeitungsverfahren zu schaffen.

Die erfindungsgemäße Aufgabe wird überraschend einfach durch die Bereitstellung von festen, exakt dosierbaren pharmazeutischen Darreichungsformen zur Einzelausgabe aus Dosiervorrichtungen gelöst, welche kleine sphäroide annähernd kugelförmige oder tropfenförmige Festkörper darstellen.

Die Erfindung wird gemäß den Ansprüchen realisiert. Die erfindungsgemäßen kleinen sphäroiden, kugelförmigen oder tropfenförmigen Festkörper, die auch andersartige Ausgestaltungen aufweisen können, weisen mindestens einen Wirkstoff zusammen mit an sich üblichen pharmazeutischen Hilfsstoffen auf und sind durch einen exakten Masse- und Wirkstoffgehalt gekennzeichnet. Sie besitzen damit hervorragende Produkteigenschaften und sind so für eine individuelle Einzeldosierung bestens geeignet.

Der besondere Vorteil besteht darin, dass diese kleinen sphäroiden Festkörper einzeln aus entsprechenden Dosiereinrichtungen, wie z. B. aus Dosierflaschen oder mittels Dosierlöffel usw., ähnlich der Tropfen-Ausgabe von flüssigen Arzneimittelzubereitungen ausgegeben werden können, wobei die Anzahl von Festkörpern bei deren Ausgabe genau bestimmt werden kann.

Des weiteren umfasst die Erfindung ein Verfahren zur Herstellung von festen sphäroiden oder eine anderweitige Form aufweisenden pharmazeutischen Darreichungsformen, das dadurch gekennzeichnet ist, dass mindestens ein Wirkstoff oder Wirkstoffkombinationen nach an sich bekannten Techniken und bei Bedarf unter Verwendung von Hilfs- und/oder Zusatzstoffen und mittels entsprechender Formwerkzeuge in die gewünschte sphäroide, annähernd kugelförmige oder tropfenförmige pharmazeutische Darreichungsform überführt wird und dass anschließend die erhaltenen Darreichungsformen zu einer durch einen Raum begrenzten Gesamtmenge zusammengefasst und vermittels Dosiervorrichtungen aus der Gesamtmenge eine vorgegebene Menge abgetrennt wird.

Die Einzeldosierung der sphäroiden Festkörper ist einfach und kann durch Abzählen oder unter Einsatz von Abteil-Hilfsmitteln für die Dosierung erfolgen. Als Abteil-Hilfsmittel können in Abhängigkeit von der Höhe der Dosierung bei einer großen Anzahl von benötigten Festkörperchen bevorzugt volumetrische Dosierhilfen, wie z. B. Messbecher, eingesetzt werden. Wird lediglich eine niedrige Dosierung des Pharmazeutikums und damit eine kleine Anzahl an Festkörperchen, beispielsweise unter 200 Applikatoren, benötigt, bietet sich zur Abteilung eine Einzellochfüllung an, wie z. B. die Verwendung eines Lochlöffels.

Erfindungsgemäß werden die sphäroiden, insbesondere mikrokleinen, Festkörper einheitlich vorzugsweise jeweils in kugel-, tropfen-, linsen-, mandel- oder zylinderähnlicher Form zur Applikation aus entsprechenden Dosiereinrichtungen bereitgestellt.

In einer bevorzugten Ausführungsvariante weisen sie einen Durchmesser von 0,5 mm bis 4 mm auf, vorzugsweise einen Durchmesser von 1,5 mm bis 3,0 mm.

Die erfindungsgemäßen kleinen, sphäroiden Festkörper werden bevorzugt zur oralen beziehungsweise peroralen Applikation in Form von an sich bekannten festen Arzneiformen dargestellt, wie z. B. in Form von Kapseln, Dragees, Tabletten, Granula, Tropfen, Globuli und/oder Pellets.

Besonders ist hierbei die tropfenförmige Ausbildung der Festkörper von Vorteil. Durch die Tropfenform erfolgt eine Masseerhöhung im bauchigen Bereich der Festkörper und somit eine Gewichtszunahme in diesem Bereich mit der Folge, dass bei einer Einzelausgabe, z. B. einer Tropfflasche, sich die so gestalteten Festkörper ausrichten und sich bei der Aufgabe nicht behindern, z. B. durch Querstellen im Bereich der Ausgabeöffnung der Tropfflasche. Des weiteren gleiten die einzelnen Festkörper aneinander vorbei in Richtung Ausgabeöffnung, wenn die Tropfflasche für eine Ausgabe von Festkörpern senkrecht oder schräg gehalten wird. Deshalb sind ganz besonders bevorzugt derartige pharmazeutische Darreichungsformen in Tropfenform geeignet, da sich diese durch die im unteren Bereich angehäufte Masse bei der Ausgabe zum Abzählen ausrichten, wodurch sich eine zusätzlich verbesserte Dosierungsmöglichkeit ergibt. Derartige Mikrotabletten weisen u. a. auch annähernd eine Kugelform auf.

Der Wirkstoffgehalt einer einzelnen Darreichungsform hängt von dem Therapie bezogenen Dosisschema ab. In Abhängigkeit vom Wirkstoff und seines Therapiegebietes werden kleine, feste, exakt dosierbare Darreichungsformen bereitgestellt, die es ermöglichen, den Wirkstoff individuell auf Art und Umfang der Behandlung sowie den Patiententyp abzustellen.

Die Herstellung der erfindungsgemäßen festen pharmazeutischen Zubereitungen erfolgt nach an sich bekannten Techniken unter Verwendung an sich üblicher Hilfs- und Zusatzstoffe je nach einzusetzendem Wirkstoff und gewünschter Darreichungsform.

Auch Herstellungsverfahren und jeweilige Anwendungsform der Wirkstoff enthaltenden Festkörper können in Abhängigkeit des(r) einzusetzenden Wirkstoffs(e) frei gewählt werden.

Die Methoden der Herstellung sind dem Fachmann im Prinzip bekannt und z. B. in Bauer, K. N.; Frömming, K.-H.; Führer, C.: Pharmazeutische Technologie, Georg-Thieme Verlag Stuttgart 1993 beschrieben.

So erfolgt in der Regel ein Vermischen von Wirkstoffen (Arzneistoffen) mit Hilfs- und/oder Zusatzstoffen. Anschließend werden die Mischungen durch Granulieren beziehungsweise Pelletieren in Granulate beziehungsweise Pellets überführt. Diese werden weiterhin gegebenenfalls zu Tabletten, Kapseln und überzogenen Arzneiformen, wie z. B. Dragees, weiterverarbeitet.

Das Vermischen von Wirk- und Hilfsstoffen kann auf an sich bekannte Weise erfolgen. Z. B. können die einzelnen Komponenten zuerst vermischt und dann aufgeschmolzen und homogenisiert werden. Je nach Empfindlichkeit des Wirkstoffs empfiehlt es sich, gegebenenfalls Hilfsstoffe zuerst aufzuschmelzen und vorzumischen und dann den Wirkstoff einzuformulieren. Misch-, Schmelz- und Formungsvorrichtungen, die auf einem Abtropf- bzw. Zwangsabtropfverfahren beruhen, sind dem Fachmann bekannt.

Weiterhin ist z. B. das Herstellen von kugelförmigen Zubereitungen durch Extrusionsformung und Sphäronisierung ein schnelles Verfahren, um kugelförmige Körper mit einheitlicher Größe und Form sowie glatter Oberfläche herzustellen. Solche kugelförmigen Pellets beziehungsweise Granulate erleichtern und verbessern sich anschließende Verfahrensschritte beispielsweise bei der Kapselabfüllung oder Tablettierung.

Das Kompaktieren der Substanzen zur Pellet- oder Tablettenherstellung kann z. B. durch Walzen- oder Matrizenkompaktierung erfolgen. Eine Tablettierung erfolgt in der Regel in speziellen Tablettiermaschinen mit Hilfe von Exzenter- und Rundlaufpressen.

Die Herstellung der erfindungsgemäßen sphäroiden Darreichungsformen kann also grundsätzlich nach allen bekannten Techniken erfolgen, die zur Herstellung fester Arzneiformen, wie z. B. von Kapseln, Dragees, Tabletten, Granula, Tropfen, Globuli und/oder Pellets, angewandt werden, wobei die Formung der jeweiligen Darreichungsformen in bevorzugte kugel-, tropfen-, linsen-, mandel- oder zylinderähnliche Form mittels entsprechender Formwerkzeuge erfolgt.

Die bevorzugte Darstellung der kleinen späroiden Festkörper in Tropfenform wird dadurch erreicht, dass man die Inhaltsstoffe nach an sich bekannten Verfahren vermischt und zu Tropfen verformt. So wird z. B. unter Verwendung von verarbeitbaren Hilfsstoffen fachgemäß eine fließfähige Masse hergestellt, diese Masse z. B. mittels rotierender Lochwerkzeuge zu Tropfen oder andere sphäroidische Partikeln verformt, welche anschließend durch Abkühlen verfestigt werden.

Entsprechende Hilfs- und Zusatzstoffe für solche Zubereitungen sind dem Fachmann bekannt. Es sind im Sinne der Erfindung alle pharmazeutisch unbedenklichen und mit dem jeweiligen ausgewählten Wirkstoff nicht negativ reagierenden Stoffe geeignet. So sind in Abhängigkeit vom Wirkstoff und vom Herstellungsverfahren alle zur Darstellung von festen pharmazeutischen Darreichungsformen an sich üblichen Hilfs- und Zusatzstoffe, wie z. B. Binde-, Füll-, Spreng- und/oder grenzflächenaktive Mittel (Netzmittel, Tenside) einsetzbar. Darüber hinaus sind alle weiteren bekannten pharmazeutischen Hilfsstoffe einsetzbar.

Dem Fachmann sind z. B. zahlreiche polymere Hilfsstoffe bekannt, beispielsweise Cellulose und -derivate, Polyvinylpyrrolidon beziehungsweise dieses enthaltende Copolymerisate oder auch Zusatzstoffe auf Basis von Acrylaten oder Methacrylate.

Als weitere Bestandteile können als Mittel hochdisperses Siliziumdioxid, Kohlenhydrate, Calcium-, Magnesium- und/oder Glycerinstearat sowie Farb-, Geschmacks- und/oder Aromastoffe, Antioxidatien und/oder Stabilisatoren enthalten sein.

Der Gehalt richtet sich nach Kriterien, die die mechanisch-physikalischen Eigenschaften der oralen Darreichungsformen bestimmen, wie z. B. Härte, Verpressbarkeit, Größe, Farbe und/oder Form.

Arzneiformen als Tabletten oder Filmtabletten können beispielsweise durch Mischen des Wirkstoffs mit bekannten Hilfsstoffen, wie Dextrose, Zucker, Cellulose, Lactose, Sorbit, Mannit, Polyvinylpyrrolidon, Sprengmitteln, wie Maisstärke oder Alginsäure, Bindemitteln, wie Stärke oder Gelatine, Gleitmitteln, wie Magnesiumstearat oder Talk und/oder Mitteln, die einen Depoteffekt erzielen können, wie Carboxylpolymethylen, Carboxymethylcellulose, Celluloseacetatphthalat oder Polyvinylacetat, erhalten werden. Die Tabletten oder Filmtabletten können auch aus mehreren Schichten bestehen. Desweiteren können Polymere hinzugefügt oder als Überzugsfilm eingesetzt werden, wie Polymethacrylsäurederivate, Celluloseacetatphthalat, HPMC, HPMC-CP, HPMC-AS. Analog können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabikum, Talk, Titandioxid oder Zucker zubereitet werden. Die Drageehülle kann aus mehreren Schichten bestehen, wobei beispielsweise die oben genannten Hilfsstoffe verwendet werden. Auch hier können Polymere hinzugefügt oder als Überzugsfilm eingesetzt werden, wie Polymethacrylsäurederivate, Celluloseacetatphthalat, HPMC, HPMC-CP, HPMC-AS. Die eingesetzten Polymere haben hier folgende Funktion: Sie sind magensaftresistent. Dadurch wird ein Schutz des Wirkstoffes vor Magensäure bzw. des Magens vor dem Wirkstoff durch eine Hülle bzw. Film um die festen Tropfen erreicht. Außerdem wird der Wirkstoff derart modifiziert freigegeben, dass eine verlängerte therapeutische Wirkung erzielt wird (Retardwirkung). Des weiteren erfolgt eine Diffusionskontrolle über eine außen um die festen Tropfen gelegte Filmmembran und eine Matrixretardierung. Zur Verbesserung des Geschmacks können Stoffe, wie Saccharin, Cyclamat oder Zucker, und/oder Aromastoffe, wie Vanillin oder Orangeextrakt, zugesetzt werden.

Die Dosierung der erfindungsgemäß ausgebildeten Festkörper erfolgt z. B. mittels Dosiervorrichtungen, mit denen es möglich ist, die Festkörper den individuellen Bedürfnissen entsprechend in variabler, frei wählbarer Anzahl zu dosieren und z. B. die entnommene Menge anhand von Markierungen in einfacher Form nach zu kontrollieren.

Nach einer ersten Ausführungsform besteht eine Dosiervorrichtung zur portionsweisen Entnahme von gleichartigen Festkörpern mittels eines in einem Spendebehältnis gekapselten, von außen zu betätigenden Schiebeteils darin, dass der Portionierungsbereich aus dem Behältnisinnern befüllt wird und nach außen entleerbar ist, wobei das Schiebeteil als Rohrstück mit einem oder mehreren Portionierungsbereichen ausgebildet ist, das durch eine äußere Umhüllung und einen neben der Auswerföffnung befindlichen, den Portionierungsbereichen zugeordneten Abstreifer geführt wird, wobei der Boden der äußeren Umhüllung kalottenförmig ausgeführt ist.

Eine weitere Dosiervorrichtung ist als Dosierlöffel ausgebildet, der einen Löffelstiel und ein Unterteil umfasst, das aus einem ebenen Mehreck besteht, das an allen Seiten mit Ausnahme an einer Seite mit einem Rand versehen ist und wobei das Unterteil mehrere Reihen von Einzelvertiefungen aufweist, die so geformt sind, dass in jeder Einzelvertiefung eine einzelne Mikrotablette passt, wo die Einzelvertiefungen zur Aufnahme der Mikrotabletten im Unterteil aus zylindrischen Bohrungen bestehen, die in schräg versetzt angeordneten Reihen in dem Unterteil ausgebildet sind, wobei der Dosierlöffel oben offen ausgebildet ist und die randfreie Seite des Unterteils parallel zur Längsachse des Löffelstiels verlaufend ist, der in Längsrichtung zu der den Rand aufweisenden Seite des Unterteils verläuft, die der randfreien Seite des Unterteils gegenüber liegend ist.

Weitere vorteilhafte Ausgestaltungen der Erfindung sind Gegenstand der Unteransprüche.

Ausführungsbeispiele der Erfindung sind in den Zeichnungen dargestellt und zwar zeigt:
- Fig. 1 und 2: vergrößerte Wiedergaben von zwei verschiedenen Ausführungsformen des erfindungssgemäßen Festkörpers in Kugel- und Tropfenform,
- Fig. 3: teils in Ansicht, teils in einem senkrechten Schnitt eine Tropfflasche,
- Fig. 4: einen Querschnitt einer ersten Ausführungsform einer Dosiervorrichtung zur portionsweisen Entnahme von Festkörpern,
- Fig. 5: einen Längsschnitt der Dosiervorrichtung gemäß Fig. 4,
- Fig. 6: eine Abwicklung des Rohrstückes der Dosiervorrichtung gemäß Fig. 4 mit einer möglichen Ausgestaltung der Portionierungsbereiche für die Festkörper,
- Fig. 7: eine weitere Ausführungsform einer Dosiervorrichtung, die als Dosierlöffel ausgebildet ist, in einer Draufsicht von oben,
- Fig. 8: einen Querschnitt durch den Dosierlöffel in der Längsrichtung gemäß Fig. 7,
- Fig. 9: den Dosierlöffel gemäß Fig. 7 in einer Ansicht von unten und
- Fig. 10: den Dosierlöffel gemäß Fig. 7 in einem Querschnitt in der Querrichtung.

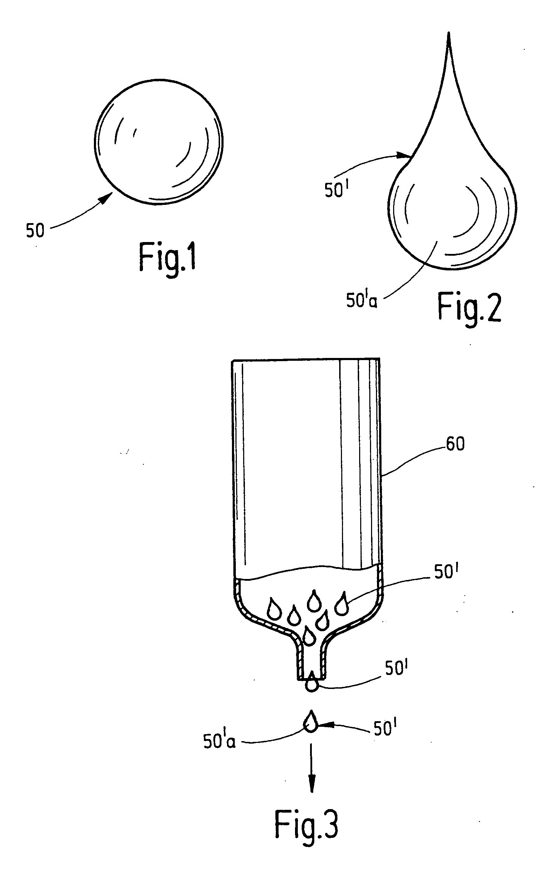

Die erfindungsgemäß ausgebildeten Festkörper 50, 50' weisen gemäß Fig. 1 eine kugelförmige Formgebung und gemäß Fig. 2 eine Tropfenform auf.

Fig. 3 zeigt eine Tropfenflasche 60 zur Ausgabe von tropfenförmigen Festkörpern 50', die sich aufgrund ihrer Ausgestaltung wie dargestellt ausrichten, was dadurch möglich ist, dass sich die Hauptmasse der Wirkstoffsubstanz im bauchigen Bereich 50'a befindet.

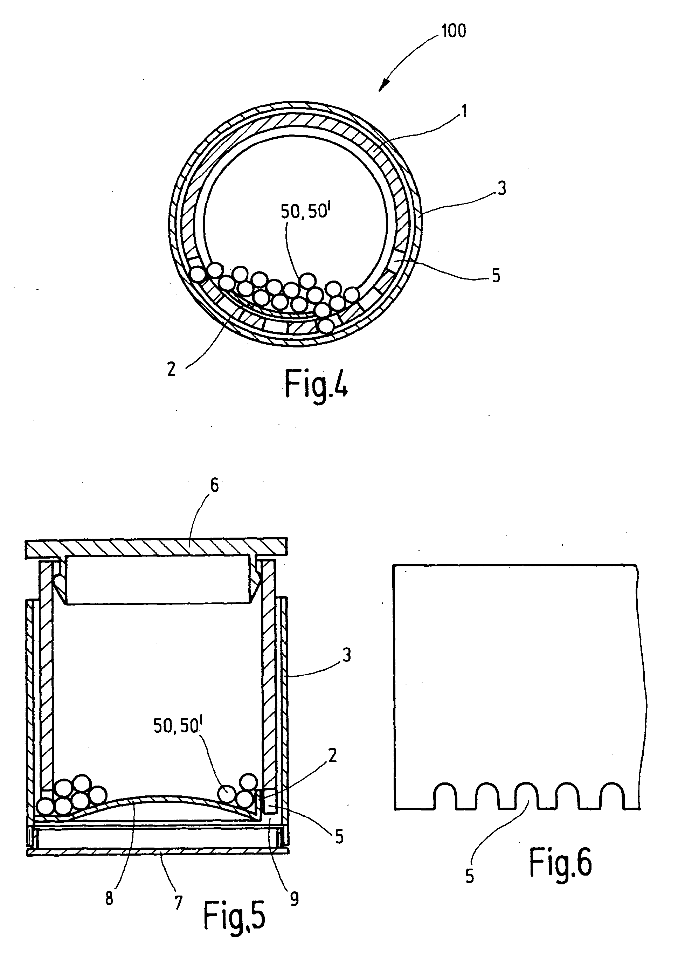

Eine portionierte Ausgabe von Festkörpern 50, 50' kann auch mittels den in den Fig. 4 bis 10 dargestellten Dosiervorrichtungen 100 vorgenommen werden.

Die Dosiervorrichtung 100 gemäß Fig. 4 umfasst einen Dosierbehälter, der gleichartige stückige Festkörper 50 enthält. Zwischen der äußeren Umhüllung 3 und dem Abstreifer 2 des Dosierbehälters ist ein Rohrstück 1 angeordnet, das seinerseits Portionierungsbereiche 5 für die Festkörper aufweist.

Die Befüllung der Portionierungsbereiche 5 geschieht aus dem Innenbereich des Dosierbehälters in dem Bereich, der nicht von dem Abstreifer 2 abgedeckt wird, durch den die Festkörper 50, 50' in den Portionierungsbereich 5 des Rohrstückes 1 gelangen.

Durch Drehen des Rohrstückes 1 mittels einer Betätigungseinrichtung 6 wird der jeweils befüllte Portionierungsbereich 5 durch den Abstreifer 2 vom Innenraum des Dosierbehälters getrennt und der Auswurföffnung 9 im Boden 8 der äußeren Umhüllung 3 des Dosierbehälters zugeführt.

Die Auswurföffnung 9 kann beispielsweise durch einen Verschlussdeckel 7, ausgestaltet als Auffangbehältnis, abgedeckt werden. Die Betätigungseinrichtung 6 dient als Verschluss für den Innenraum des Dosierbehältnisses. Die Markierungen für die Portionierungsmengen werden von außen sichtbar auf dem Rohrstück 1 aufgebracht.

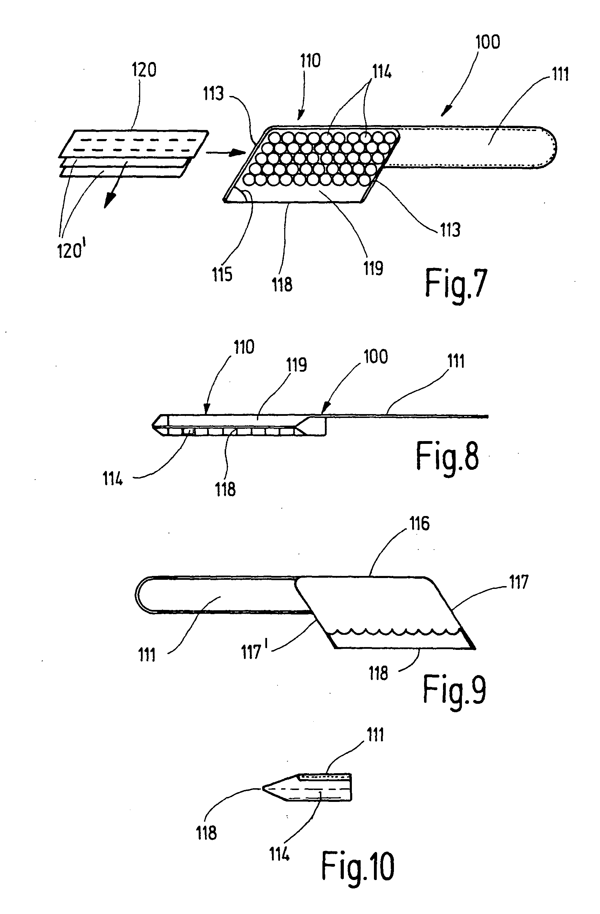

Eine weitere Ausführungsform einer Dosiervorrichtung 100 zeigen die Fig. 7 bis 10.

Hiernach besteht die Dosiervorrichtung aus einem Dosierlöffel 110, der darin besteht, dass die Einzelvertiefungen zur Aufnahme der Festkörper im Unterteil des oben offen ausgebildeten Dosierlöffels aus zylindrischen Bohrungen bestehen, die in schräg versetzt angeordneten Reihen in dem Unterteil ausgebildet sind und dass die randfreie Seite des Unterteils parallel zur Längsachse des Löffelstiels verlaufend ist, der in Längsrichtung zu der den Rand aufweisenden Seite des Unterteils verläuft, die der randfreien Seite des Unterteils gegenüberliegend ist. Gegenstand ist somit ein Dosierlöffel für Festkörper, bei dem das Unterteil 112 des Löffels aus einem ebenen Mehreck besteht, welches an allen Seiten mit Ausnahme an einer Seite einen Rand 113 besitzt und wobei das Mehreck eine Reihe von Einzelvertiefungen 114 aufweist, die so geformt sind, dass in jede Einzelvertiefung ein einzelner Festkörper passt bzw. aufgenommen wird.

Das Mehreck ist in der Regel ein Viereck, bei dem die zwei Seiten, die sich gegenüber liegen, dieselbe Länge haben (Parallelogramm). Der kleine Winkel 115 des Parallelogramms liegt zwischen 45° und 90°. Eine lange Seite 116 des Parallelogramms sowie die beiden kleineren Seiten 117, 117' sind mit einem Rand 113 versehen, der etwas, d. h. bis zu 5 mm, senkrecht über das Mehreck hinausragt.

In das Mehreck des Dosierlöffels sind kleine zylindrische Bohrungen 114 eingelassen, deren Durchmesser und Tiefe so bemessen ist, dass ein Festkörper in jede Öffnung bequem hineinpasst. Der Durchmesser der Bohrungen liegt zwischen 1,5 und 4,0 mm. Dasselbe gilt für die Tiefe der Bohrungen. Im speziellen Fall sollten Durchmesser und Tiefe 0,2 mm größer sein als die größte Diagonale der Mikrotablette, für die der Dosierlöffel verwendet werden soll. Die zylindrischen Bohrungen sind normalerweise so angeordnet, dass möglichst viele Löcher auf 1 cm² des Mehrecks passen. Die Gesamtzahl der Löcher entspricht der Menge an einzunehmenden Festkörpern. Diese Zahl liegt in der Regel bei 5 bis 100, vorzugsweise 10 bis 60.

An der randlosen Seite 118 des Vierecks liegt zweckmäßig noch eine Zone ohne Öffnungen 119, die normalerweise bis zu 1 cm breit sind. Diese Zone erleichtert das Füllen der Löcher mit Festkörpern, besonders aus einem Behältnis heraus, das nur noch geringe Mengen an Festkörpern enthält.

Der Löffelstiel 110 ist vorzugsweise in Verlängerung der Seite am längsten Rand angebracht.

Um die von dem Dosierlöffel 110 aufzunehmende Menge bzw. Anzahl von Festkörpern 50, 50' vorgeben zu können, kann das Unterteil 112 des Dosierlöffels 110 mit einem an den Seitenrändern 113 geführten Schieber 120 versehen sein, um abschnittsweise die mit den Bohrungen 114 versehene Fläche abschnittsweise abdecken bzw. verschließen zu können, so dass z. B. nur zwei Reihen Bohrungen 114 zur Aufnahme von Festkörpern 50, 50' zur Verfügung stehen. Auf diese Weise ist eine genaue Menge an Festkörpern abteilbar. Dieser Schieber 120 kann z. B. aus einer Anzahl von ineinander schiebbaren Abschnitten 120a bestehen oder jalousieartig ausgebildet sein (Fig. 7).

Auch andersartig ausgestaltete Dosiervorrichtungen für die Festkörper können zur Anwendung gelangen.

Die vorangehend beschriebenen Dosiervorrichtungen stellen bevorzugte Ausführungsbeispiele dar.

## Patentansprüche

1. Feste, exakt dosierbare pharmazeutische Darreichungsformen zur Einzelausgabe aus Dosiervorrichtungen, wobei die Darreichungsformen kleine sphäroide Festkörper mit mindestens einem Wirkstoff zusammen mit pharmazeutischen Hilfsstoffen darstellen und einen exakten Masse- und Wirkstoffgehalt aufweisen.

2. Darreichungsform nach Anspruch 1,
**dadurch gekennzeichnet,**
**dass** die sphäroiden Festkörper jeweils in kugel-, tropfen-, linsen-, mandel- oder zylinderähnlicher Form vorliegen.

3. Darreichungsform nach Anspruch 2,
**dadurch gekennzeichnet,**
**dass** die sphäroiden Festkörper in Tropfenform vorliegen.

4. Darreichungsform nach einem der Ansprüche 1 bis 3,
**dadurch gekennzeichnet,**
**dass** die sphäroiden Festkörper einen Durchmesser von 0,5 mm bis 4 mm aufweisen.

5. Darreichungsform nach Anspruch 4,
**dadurch gekennzeichnet,**
**dass** die sphäroiden Festkörper einen Durchmesser von 1,5 mm bis 3,0 mm aufweisen.

6. Darreichungsform nach einem der Ansprüche 1 bis 5,
**dadurch gekennzeichnet,**
**dass** die sphäroiden Festkörper als Dragees, Tabletten, Filmtabletten, Granula, Tropfen im festen Aggregatzustand und/oder Pellets vorliegen.

7. Darreichungsform nach einem der Ansprüche 1 bis 6,
**dadurch gekennzeichnet,**
**dass** die sphäroiden Festkörper mit einer Umhüllung aus einem Polymer versehen sind.

8. Verfahren zur Herstellung von festen sphäroiden oder eine anderweitige Form aufweisenden pharmazeutischen Darreichungsformen nach einem der Ansprüche 1 bis 7,
**dadurch gekennzeichnet,**
**dass** mindestens ein Wirkstoff oder Wirkstoffkombinationen nach an sich bekannten Techniken und bei Bedarf unter Verwendung von Hilfs- und/oder Zusatzstoffen und mittels entsprechender Formwerkzeuge in die gewünschte sphäroide, annähernd kugelförmige oder tropfenförmige pharmazeutische Darreichungsform überführt wird und dass anschließend die erhaltenen Darreichungsformen zu einer durch einen Raum begrenzten Gesamtmenge zusammengefasst und vermittels Dosiervorrichtungen (100) aus der Gesamtmenge eine vorgegebene Menge abgetrennt wird.

9. Verfahren nach Anspruch 8 zur Herstellung von tropfenförmigen Darreichungsformen,
**dadurch gekennzeichnet,**
**dass** die Inhaltsstoffe vermischt und zu Tropfen in festem Aggregatzustand verformt werden.

10. Dosiervorrichtung zur portionsweisen Entnahme von gleichartigen Darreichungsformen (4) nach einem der Ansprüche 1 bis 9 mittels eines in einem Spendebehältnis gekapselten, von außen zu betätigenden Schiebeteils, dessen Portionierungsbereich (5) aus dem Behältnisinnern befüllt wird und nach außen entleerbar ist, wobei das Schiebeteil als Rohrstück (1) mit einem oder mehreren Portionierungsbereichen (5) ausgebildet ist, das durch eine äußere Umhüllung (3) und einem neben der Auswerföffnung (9) befindlichen, den Portionierungsbereichen (5) zugeordneten Abstreifer (2) geführt wird, wobei der Boden (8) der äußeren Umhüllung (3) kalottenförmig ausgeführt ist.

11. Vorrichtung nach Anspruch 10,
**dadurch gekennzeichnet,**
**dass** die Betätigungseinrichtung (6) für das Rohrstück (1) als Verschluss der Befüllöffnung dient.

12. Vorrichtung nach Anspruch 10 und 11,
**dadurch gekennzeichnet,**
**dass** an das Unterteil der äußeren Umhüllung (3) ein Verschlussdeckel (7) zur Aufnahme der portionierten stückigen Güter (4) befestigt wird.

13. Vorrichtung nach Anspruch 10 bis 12,
**dadurch gekennzeichnet,**
**dass** Markierungen für die Portionierungsmengen vorgesehen sind.

14. Dosiervorrichtung zur portionsweisen Entnahme von gleichartigen Darreichungsformen (4) nach einem der Ansprüche 1 bis 9,
**dadurch gekennzeichnet,**
**dass** die Dosiervorrichtung (100) als Dosierlöffel (110) mit einem Löffelstiel (111) und mit einem Unterteil (112) ausgebildet ist, das aus einem ebenen Mehreck besteht, das an allen Seiten mit Ausnahme an einer Seite mit einem Rand (113) versehen ist, und wobei das Unterteil (112) mehrere Reihen von Einzelvertiefungen aufweist, die so geformt sind, dass in jeder Einzelvertiefung ein einzelner Formkörper passt, wo die Einzelvertiefungen zur Aufnahme der Formkörper im Unterteil (112) aus zylindrischen Bohrungen (114) bestehen, die in schräg versetzt angeordneten Reihen in dem Unterteil (112) ausgebildet sind, wobei der Dosierlöffel oben offen ausgebildet ist und die randfreie Seite (118) des Unterteils (112) parallel zur Längsachse des Löffelstiels (111) verlaufend ist, der in Längsrichtung zu der den Rand aufweisenden Seite des Unterteils (112) verläuft, die der randfreifen Seite (118) des Unterteils (112) gegenüber liegend ist.

15. Dosiervorrichtung nach Anspruch 14,
**dadurch gekennzeichnet,**
**dass** das Mehreck des Unterteils (112) aus einem Viereck in Form eines Parallelogramms besteht, bei dem die sich gegenüberliegenden Seiten dieselben Längen aufweisen, wobei bevorzugterweise der kleine Winkel (115) zwischen zwei Seiten des Parallelogramms zwischen 45° und 90° liegt, und wobei eine lange Seite (116) des Parallelogramms sowie die beiden kurzen Seiten (117, 117') mit einem Rand (113) versehen sind.

16. Dosiiervorrichtung nach Anspruch 14 und 15,
**dadurch gekennzeichnet,**
**dass** der Rand (113) der beiden kürzeren Seiten (117, 117') des Parallelogramms geringfügig, beispielsweise bis zu 5 mm senkrecht über das Mehreck hinausragt.

17. Dosiervorrichtung nach einem der Ansprüche 14 bis 16,
**dadurch gekennzeichnet,**
**dass** der Durchmesser und die Tiefe der zylindrischen Bohrung (114) größer sind als die größte Diagonale einer Mikrotablette.

18. Dosiervorrichtung nach einem der Ansprüche 14 bis 17,
**dadurch gekennzeichnet,**
**dass** der Durchmesser und die Tiefe der zylindrischen Bohrung (114) zwischen 1,5 mm und 4,0 mm liegen.

19. Dosiervorrichtung nach einem der Ansprüche 14 bis 18,
**dadurch gekennzeichnet,**
**dass** die Zuordnung der zylindrischen Bohrungen (114) in dem Unterteil (112) dicht bei dicht ist.

20. Dosiervorrichtung nach einem der Ansprüche 14 bis 19,
**dadurch gekennzeichnet,**
**dass** die Gesamtzahl der zylindrischen Bohrungen (114) in dem Unterteil (112) der Menge der aufzunehmenden Festkörper entspricht, wobei die Anzahl der zylindrischen Bohrungen (114) bei 5 bis 100, vorzugsweise 10 bis 60 liegt.

21. Dosiervorrichtung nach einem der Ansprüche 14 bis 20,
**dadurch gekennzeichnet,**
**dass** an der randlosen Seite (118) des Mehrecks zum erleichterten Befüllen der zylindrischen Bohrungen mit Mikrokapseln eine bohrungsfreie Zone ausgebildet ist, die bevorzugterweise bis zu 1 cm breit ist.

22. Dosiervorrichtung nach einem der Ansprüche 14 bis 21,
**dadurch gekennzeichnet,**
**dass** die Bohrungen (114) in dem Unterteil (112) des Dosierlöffels (110) vermittels eines an den Seitenrändern (113) des Unterteils (112) geführten Schiebers (120) abschnittsweise verschließbar sind.

23. Verwendung von kleinen sphäroiden oder tropfenförmigen Festkörpern, die mindestens einen Wirkstoff oder eine Wirkstoffkombination zusammen mit pharmazeutischen Hilfsstoffen umfassen und die einen exakten Masse- und Wirkstoffgehalt aufweisen, in Dosiervorrichtungen (100) für eine auf einen Patienten abgestimmte individuelle Einzeldosierung.

24. Verwendung nach Anspruch 23,
**dadurch gekennzeichnet,**
**dass** die sphäroiden Festkörper jeweils in kugel-, tropfen-, linsen-, mandel- oder zylinderähnlicher Form, vorzugsweise in Tropfenform, vorliegen.

25. Verwendung nach Anspruch 23 oder 24,
**dadurch gekennzeichnet,**
**dass** die sphäroiden Festkörper einen Durchmesser von 0,5 mm bis 4 mm, vorzugsweise 1,5 mm bis 3,0 mm, aufweisen.

26. Verwendung nach einem der Ansprüche 23 bis 25,
**dadurch gekennzeichnet,**
**dass** die Einzeldosierung der sphäroiden Festkörper durch Abzählen oder unter Einsatz von Abteil-Hilfsmitteln für die Dosierung erfolgt.

27. Verwendung nach einem der Ansprüche 23 bis 26,
**dadurch gekennzeichnet,**
**dass** als Abteil-Hilfsmittel in Abhängigkeit von der Höhe der Dosierung volumetrische Dosierhilfen oder Dosiervorrichtungen zur Einzellochfüllung eingesetzt werden.
